# EUROPEAN PATENT APPLICATION

(11) **EP 4 129 365 A1**
(43) Date of publication of application: **08.02.2023**
(21) Application number: 21782115.6
(22) Date of filing: 31.03.2021
(51) Int. Cl.: A61M 5/315, A61M 5/50

(54) **MEDICAL SYRINGE PLUNGER AND MEDICAL FINGER GRIP**

(30) Priority: 31.03.2020 JP 2020062639
(71) Applicant: Daikyo Seiko, LTD., Sano-shi, Tochigi 327-0813 (JP)
(72) Inventor: YOSHIDA Takayuki, Sano-shi, Tochigi 327-0813 (JP)
(74) Representative: Grünecker Patent- und Rechtsanwälte PartG mbB
(86) International application number: PCT/JP2021/013776
(87) International publication number: WO 2021/201075

(57) **Abstract**

**Problem**

To provide a medical syringe plunger and a medical finger grip for a disposal syringe having both a dose accuracy function and a plunger pulling out prevention function.

**Solution**

A plunger rod includes a first guide groove for guiding the axial movement by a protrusion of a finger grip which extends toward the center of the hole of the finger grip and moves in it, a second guide groove being formed at a position on the plunger rod which is rotated by a predetermined angle in the circumferential direction with respect to the first guide groove for guiding the axial movement, an axial direction movement restriction section being formed in the vicinity of the end point of the first guide groove for restricting the protrusion's movement in the axial direction, and a protrusion transition section for enabling the protrusion to transit from the first guide groove to the second guide groove.

## Description

### Technical Field

The present invention relates to a syringe plunger and a finger grip for constituting a medical syringe.

### Background Art

In general, a medical syringe comprises a syringe barrel having a tip to which an injection needle is to be attached and a syringe plunger which is to be inserted into the syringe barrel from an opening at the other end for moving a piston in the axial direction. A flange section is formed at the opening on the other end of the syringe barrel to project radially outward. In such a syringe, a back stop function is required to prevent the plunger from coming out the barrel unintentionally after the tip of the plunger is inserted in the barrel. It is also required to have a function for discharging a predetermined amount of a medical solution from the tip of the syringe at the time of injection.

For example, Patent Document 1 proposes a syringe including a plunger which has a shape like saw teeth with plural pits and a complicated mechanism installed in a finger wing which is attached to a flange. Patent Document 2 discloses a syringe including a plunger having two shaft members and a complicated structure for ejecting small portions of the content.
Patent Document 1: US Laid Open Patent Application 2019111211A1
Patent Document 2: Japanese Laid Open Patent Application 2017-80189

### Disclosure of invention

### Problems to be resolved by the Invention

For simplifying medicine management, controlling infection, preventing errors at the time of dispensing, speeding up administration and so on, a prefilled syringe, in which an accurate dosage of medicine is previously filled, is used. For example, even in a minimal capacity syringe for ophthalmic use, it is required to have a dose accuracy (accuracy of filling and administration) function for filling a barrel with a predetermined amount of an internal-use solution accurately by a simple operation. It is also required to have a back stop function for preventing a plunger from being pulled out a syringe barrel to ensure prevention of re-use of a used syringe after injection in view of medical safety like prevention of infection.

Therefore, it is required to provide a minimal capacity syringe as a disposable product, for which a single time use is assumed, having both a dose accuracy function and a plunger pulling out prevention function without requiring a complicated mechanism.

### Means for solving the Problems

(1)A medical syringe plunger according to the present invention comprises a plunger rod and a plunger head which is formed at the end edge of the plunger, for using by being inserted in a hole of a finger grip which is attached to a flange section of a syringe barrel.

The plunger rod includes a first guide groove and a second guide groove. The first guide groove is formed in a range of a first predetermined length in the axial direction from the tip. The axial movement of the plunger rod is guided by a protrusion of a finger grip which moves in the first guide groove. The protrusion is formed to extend toward the center of the hole of the finger grip.

The second guide groove is formed in a range of a second predetermined length in the axial direction at a position on the plunger rod which is rotated a predetermined angle in the circumferential direction with respect to the first guide groove. The axial movement of the plunger rod is guided by a protrusion of a finger grip which moves in the second guide groove. The protrusion is formed to extend toward the center of the hole of the finger grip.

By configuration as described above, the protrusion of the finger grip can move in the first guide groove from the tip of the plunger rod toward the end edge for the first predetermined length in the axial direction. A predetermined amount of the internal-use solution can be held in the syringe barrel by putting the internal-use solution and the piston in the syringe barrel, pushing the piston with the tip of the plunger rod and moving the protrusion of the finger grip in the first guide groove toward the end edge in the axial direction by the first predetermine length.

In one embodiment of the present invention, it is also possible to form a third guide groove on the extension line of the first guide groove and set the distance in which the plunger rod can be pushed into the syringe barrel to a sum of the first predetermined length and the third predetermined length. In this case, it is also possible to hold a predetermined amount of the internal-use solution in the syringe barrel by pushing the piston with the tip of the plunger rod.

It is possible to set so as to administer the total amount of the internal-use solution by rotating the protrusion of the finger grip by a predetermined angle in the circumferential direction around the plunger rod, moving the protrusion to the second guide groove, pushing the plunger rod further and moving the protrusion by the second predetermined length.

(2)In a medical syringe plunger described in (1) according to the present invention, it is possible to form a protrusion transition section between the first guide groove and the second guide groove for enabling the protrusion to move from the first guide groove to the second guide groove.

In one embodiment of the present invention, a third guide groove is formed on the extension line of the first guide groove in a range of a third predetermined length from the end point of the first guide groove. The second guide groove has a start point formed at a position which is not closer to the tip side than the end point of the first guide groove and an end point formed at the side of the end edge of the plunger rod. A partition wall having a thinner cross section at the upper part than the cross section of the lower part is formed between at least a portion of a side surface of the third guide groove and a side surface of the start point part of the second guide groove. Such a partition wall is formed as a protrusion transition section.

Even if it is attempted to push the plunger head in the syringe barrel during the time when the protrusion is positioned in the third guide groove, the plunger rod cannot move any further in the axial direction when the protrusion reaches the end point of the third guide groove, and no internal-use solution will be ejected from the tip of the syringe barrel.

It becomes possible to move the plunger rod in the axial direction by rotating the plunger head and the plunger rod by a predetermined angle, having the protrusion ride over the partition wall and putting the protrusion in the second guide groove. It is possible to administer the total amount of the internal-use solution by pushing the plunger rod further and moving the protrusion by the second predetermined length. It becomes possible for the protrusion to ride over the partition wall by forming the partition wall having a width which is equal to or slightly larger than the thickness of the protrusion between at least a portion of the side surface of the third guide groove and the side surface of the start point part of the second guide groove. As the protrusion transition section is formed as a partition wall having a thinner cross section at the upper part in comparison with the cross section at the lower part, namely a partition wall having an angular cross section in which the upper part is thin, it becomes easy for the protrusion to ride over the partition wall.

(3)In a medical syringe plunger according to the present invention described in (1) or (2), it is possible to form an axial direction movement restriction section which allows a protrusion to move toward the plunger head until the end point of the first guide groove but restricts the movement of the protrusion in the axial direction on the extension line of the first guide groove when the protrusion passed over the end point. It is possible for the second guide groove to have a start point formed at a position which is not closer to the tip side of the plunger rod than the position of the axial direction movement restriction section and an end point formed at the end edge side of the plunger rod.

In one embodiment of the present invention, the axial direction movement restriction section is the start point of the third guide groove which is formed on the extension line of the first guide groove, and it is also the end point (the upright wall) of the first guide groove in which the vicinity of the end point is formed in a wedge shape. After the protrusion of the finger grip passed over the end point of the first guide groove, the protrusion becomes able to move in the axial direction only within the short axial length (the third predetermined length) of the third guide groove.

It is arranged so that a predetermined amount of the internal-use solution is held in the syringe barrel when the protrusion of the finger grip reaches the end point of the third guide groove. The amount of the internal-use solution filled in the syringe is determined in relation with the inner diameter of the intermediate body part of the syringe barrel and the tip position of the plunger rod which is fixed when the protrusion reaches the end point of the third guide groove which are used as a combination. Therefore, a predetermined amount of the internal-use solution is filled in the syringe barrel accurately by the operation of simply pushing the plunger rod into the syringe barrel when the first predetermined length until the end point of the first guide groove and the third predetermined length until the end point of the third guide groove are set appropriately depending on the syringe barrel which is to be used.

The second guide groove has a start point formed at a position which is not closer to the tip side than the axial direction movement restriction section (the end point of the first guide groove) and an end point formed at the end edge side of the plunger rod. The total amount of the internal-use solution in the syringe barrel is administered certainly by the operation of simply pushing the plunger rod into the syringe barrel.

It is impossible to pull the syringe plunger out the syringe barrel after the administration of the internal-use solution because the protrusion of the finger grip cannot move to the position which is closer to the tip side of the plunger rod than the axial direction movement restriction section (the end point of the first guide groove)

(4) A medical syringe plunger according to the present invention comprises a plunger rod and a plunger head which is formed at the end edge of the plunger rod, and it is for using by being inserted into a hole of a finger grip which is to be attached to the flange section of the syringe barrel. The plunger rod has a first guide groove which is formed in a range of a first predetermined length in the axial direction from the tip. The first guide groove is formed for guiding the axial movement by a protrusion which moves in the first guide groove. The protrusion is formed to extend toward the center of the hole of the finger grip.
The first guide groove is formed in a wedge shape having an inclined surface which goes toward the surface of the plunger rod from the bottom of the first guide groove in a range from a point before the end point to the end point, and the end point is formed as an upright wall. The first guide groove is formed in a wedge shape having a wedge-shaped cross section in the vicinity of the end point of the first guide groove.

When a medical syringe plunger configured as described above is inserted into the hole of the finger grip which is attached to the flange section of the syringe barrel, it prevents the protrusion from moving toward the reverse direction in the axial direction by the upright wall when the tip of the protrusion of the finger grip rides over the end point of the first guide groove while being pushed against the inclined surface of the wedge shape. By this operation, the tip position of the plunger rod is fixed inside the syringe barrel.

In one embodiment of the present invention, the third guide groove is formed in a range of the third predetermined length from the end point of the first guide groove. It becomes impossible to push the plunger rod into the syringe barrel any further when the protrusion of the finger grip reaches the end point of the third guide groove by pushing the plunger rod into the syringe barrel. By this operation, it is possible to hold a predetermined amount of the internal-use solution in the syringe barrel. It prevents the plunger rod from being pulled out the hole of the finger grip, because the protrusion of the finger grip cannot move in the reverse direction by the end point (the upright wall) of the first guide groove.

(5) A medical finger grip according to the present invention has a shape for being attached to cover at least a portion of the flange section of a syringe barrel. A flat plate section for covering an opening of the flange section has a hole through which a plunger rod passes, and the flat plate section has a protrusion extending toward the center of the hole. The protrusion has a length reaching a predetermined depth of the guide groove which is formed in the plunger rod. The protrusion is formed so that the tip is bent when it rides over a structure formed in the guide groove like a wedge-shaped inclined surface or a partition wall as examples, and the protrusion returns to the original shape after it rides over the structure.

When the plunger rod is inserted in the hole of the medical finger grip configured as described above for use, the protrusion moves inside the guide groove and guides the axial movement of the syringe plunger. If the wedge shape section is formed in a guide groove, when the protrusion moves toward the surface of the plunger rod from the bottom of the guide groove along the inclined surface of the wedge shape section, the tip is bent by being pushed against the inclined surface, and it returns to the original shape after passing the wedge shape section. It prevents the plunger rod from moving in the reverse direction by the protrusion when the end point of the wedge shape section is formed as an upright wall. When the first guide groove and the second guide groove are formed in the plunger rod, and a partition wall is formed between the first guide groove and the second guide groove, the tip of the protrusion is bent by being pushed against the partition wall and it returns to the original shape after passing the partition wall. By this operation, the protrusion transits from the first guide groove to the second guide groove.

### Effect of Invention

In a medical syringe plunger and a medical finger grip according to the present invention, it is possible to provide a disposal syringe, for which a single time use is assumed, which can fill and administer a predetermined amount of an internal-use solution and prevent the syringe plunger from pulling out the syringe barrel after the use.

### Brief Description of Drawings

Fig. 1 (A) shows a perspective view of a configuration example of a medical syringe made by assembling a syringe plunger and a finger grip according to one embodiment of the present invention together with a syringe barrel, and (B) shows a perspective view of a medical syringe shown in (A) which is observed from a different angle for showing a notched portion of the finger grip and the attachment to the flange section in detail.
Fig.2 shows an axial cross-sectional view of the syringe barrel shown in Fig. 1.
Fig. 3 shows a configuration a finger grip according to one embodiment of the present invention, shown in Fig. 1, (A) shows a perspective view, (B) shows a top view, (C) shows a side view observed from the direction shown by the arrow S in (B), and (D) shows a bottom view.
Fig. 4 shows a side view of a configuration of a syringe plunger according to one embodiment of the present invention.
Fig. 5 shows a cross sectional view of a syringe plunger shown in Fig. 4, (A) shows an A-A' cross sectional view observed from the direction of the tip of the syringe plunger, (B) shows a B-B' cross sectional view observed from the direction of the tip of the syringe plunger, and (C) shows a C-C' cross sectional view.
Fig. 6 (A) shows a perspective view of a configuration of a portion of the plunger rod shown in Fig.4, (B) shows an enlarged view of the B portion surrounded by the dotted line in (A).
Fig. 7 shows a partial enlarged perspective view of a medical syringe shown in Fig. 1 for explaining the configuration and operation of a syringe plunger according to one embodiment of the present invention.

### A Mode for implementing the Invention

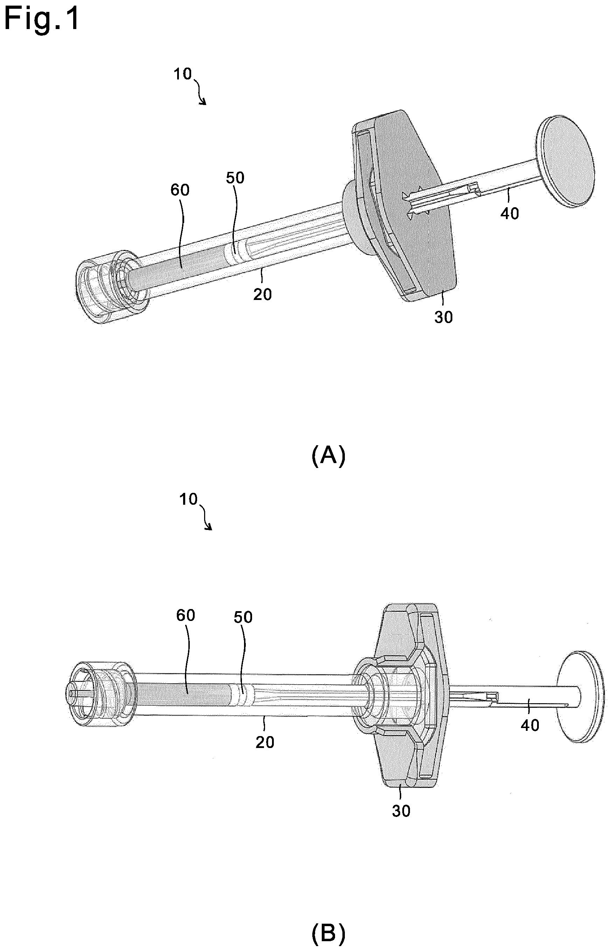

A medical syringe plunger and a medical finger grip according to one embodiment of the present invention will be explained below referring to the drawings. Fig.1 shows a configuration example of an assembly of the syringe plunger and the finger grip according to one embodiment of the present invention together with a syringe barrel as a medical syringe.
This medical syringe is a minimal capacity syringe for ophthalmic use as an example and a disposable syringe for which a single time use is assumed.

In Fig.1, a syringe 10 is an assembly of a syringe barrel 20, a finger grip 30, a syringe plunger 40 and a piston 50. An internal-use solution 60 is pushed out from the tip of the syringe barrel 20 by accommodating the internal-use solution 60 in the hollow barrel section of the tubular syringe barrel 20 and pushing the piston 50 with the tip of the syringe plunger 40. As shown in Fig.1, the syringe barrel 20 is a transparent barrel of a molded article made of a polyolefin resin material as an example, and it is possible to visually recognize the internal-use solution 60, the piston 50 and a part of the syringe plunger 40 which are inside the hollow barrel section.

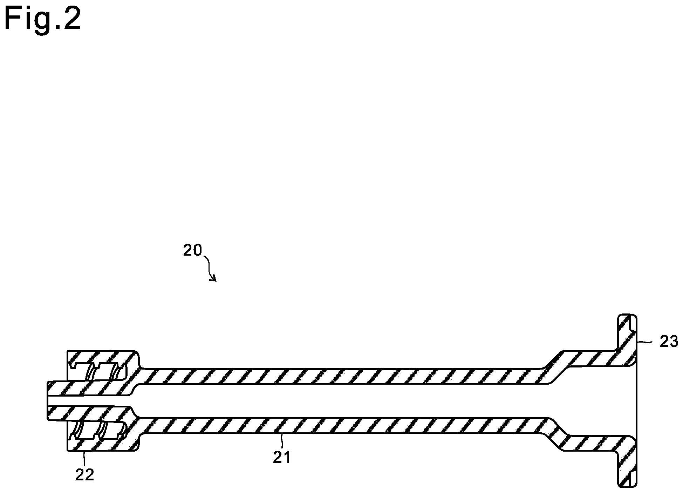

Fig. 2 is an axial sectional view which shows a configuration of the syringe barrel 20. The syringe barrel 20 comprises a tubular barrel section 21 for accommodating an internal-use solution in the interior thereof, an injection nozzle section 22 which is formed at one end of the barrel section 21, and a flange section 23 which is formed at an opening on the other end of the barrel section 21 to project radially outward. An injection needle may be attached to the injection nozzle section 22, and the injection nozzle section 22 may be also used for mixed injection to a drip infusion portion, a two-liquid blending syringe between syringes or the like.

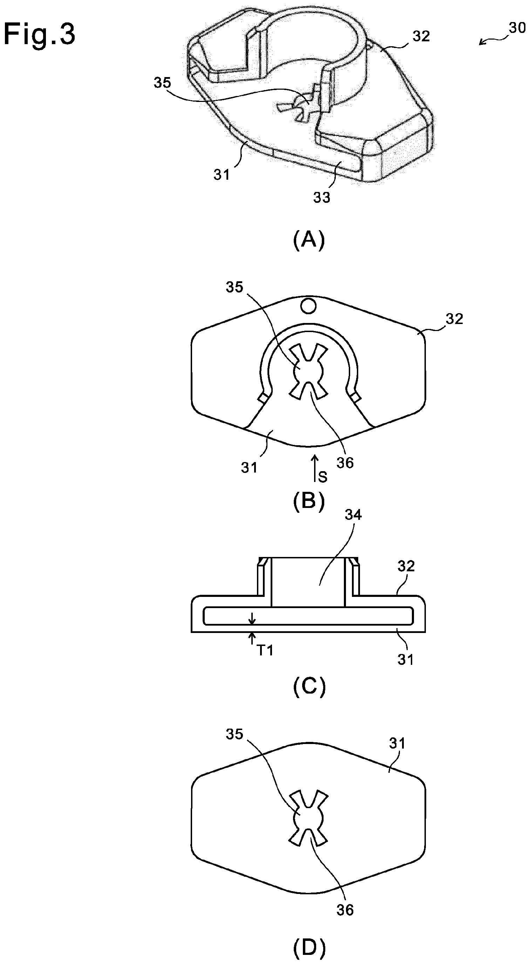

In a minimal capacity syringe, a finger grip is attached to the flange section 23 for improving the operation by a medical worker as an example because the outer diameter of the flange section 23 is small. Fig.3 shows a configuration of a finger grip 30 according to one embodiment, (A) shows a perspective view, (B) shows a top view, (C) shows a side view observed from the direction shown by the arrow S in (B), and (D) shows a bottom view.

In Fig.3, the finger grip 30 comprises a flat plate section 31 for covering an opening of the flange section 23 of the syringe barrel 20 and a cover section 32 for covering the flange section 23 except for a portion. The cover section 32 has a notched portion 33 for attaching to the flange section 23. As shown in Fig.3(A), (C), the finger grip 30 has a hollow structure having an internal space 34, the flange section 23 of the syringe barrel 20 and a portion of the barrel section in its vicinity are fitted in the internal space 34 as shown in Fig.1(B).

As shown in Fig.3(B), (D), the flat plate section 31 has a hole 35 for inserting a plunger rod, and the hole 35 is formed so that a tongue-shaped protrusion 36 having a thinner tip extends toward the center of the hole 35. The protrusion 36 has a length which reaches a predetermined depth in the groove formed in the plunger rod which will be described later. Therefore, as shown in Fig.3(B), (D), the hole 35 has a shape of a combination of the arc portions having a radius corresponding to the outer diameter of the plunger rod and the portions which form the protrusions 36.

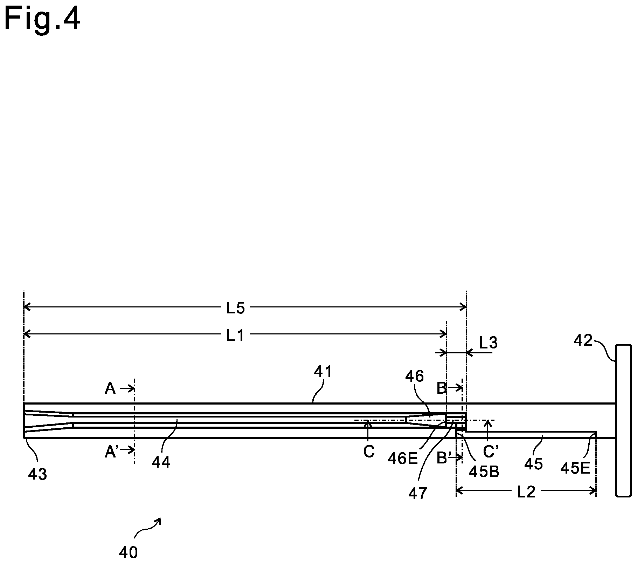

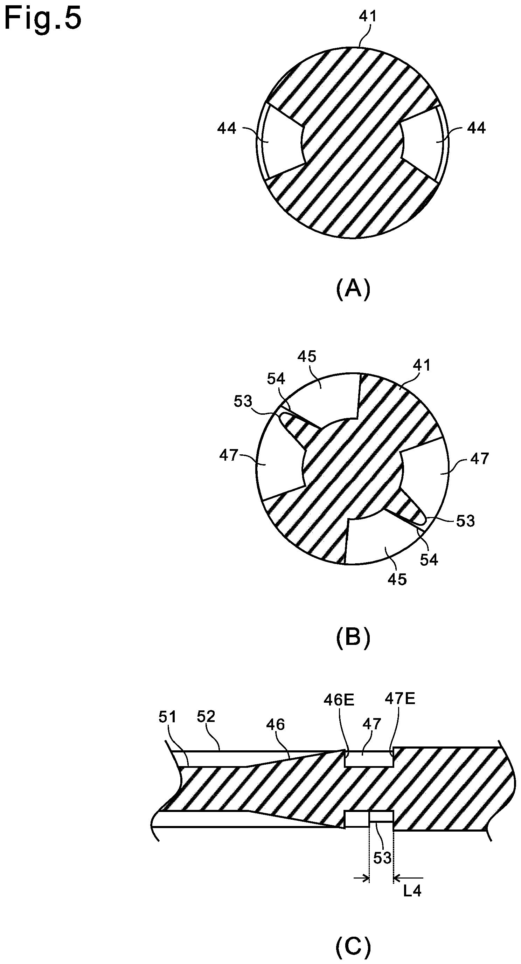

Fig.4 shows a side view of a syringe plunger 40 according to one embodiment of the present invention, Fig.5(A) shows a A-A' cross-sectional view of the syringe plunger 40 shown in Fig.4, (B) shows a B-B' cross-sectional view of the syringe plunger 40, (C) shows a C-C' cross-sectional view of the syringe plunger 40. In Fig.4, the syringe plunger 40 comprises a round bar shaped plunger rod 41 and a disc-shaped plunger head 42 which is formed at the end edge of the plunger rod 41. The plunger rod 41 has a first guide groove 44 which is formed in a range of a length L1 in the axial direction from the tip 43 and a second guide groove 45 of a length L2 at the position which is rotated by about 60 degrees in a circumferential direction of the plunger rod 41 with respect to the first guide groove 44.

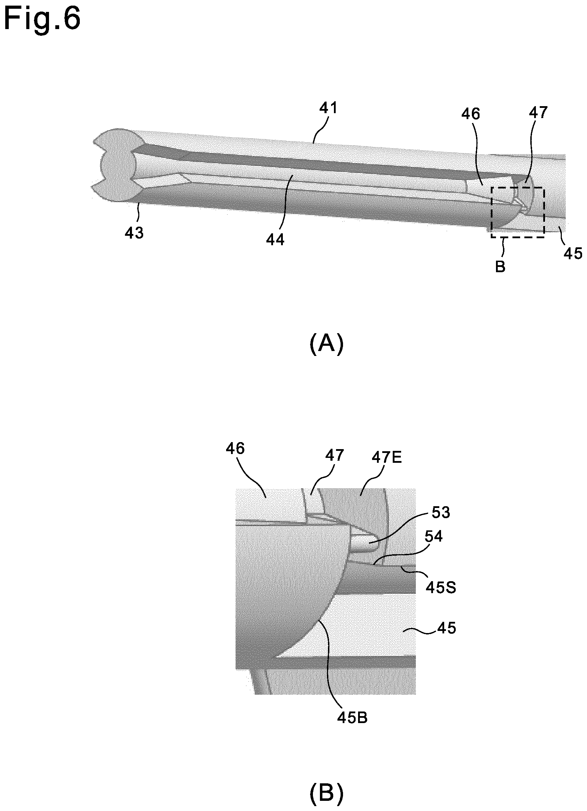

As shown in Fig.5(A), the first guide groove 44 is a groove having a fan-like cross-sectional shape in the center portion, and two first guide groove 44 are formed at the opposite positions in a circumferential direction of the plunger rod 41. As shown in Fig.4 and Fig.6(A), the first guide groove 44 has a wider width in the portion of the tip 43 of the plunger rod 41, and it has a structure in which the protrusion 36 can be easily moved in the first guide groove 44 when the plunger rod 41 is inserted into the hole 35 of the finger grip 30 in Fig.3. As the length of the arc portion having a radius corresponding to the outer diameter of the plunger rod 41 is set to be gradually shorter toward the end edge from the tip 43 in a range of a predetermined length, it is arranged to restrict the movement of the protrusion 36 in the circumferential direction and guide the movement of the plunger rod 41 in the axial direction and the rotational direction by the protrusion 36. The fan-like cross-sectional shape of the first guide groove 44 is suitable for guiding the movement of the plunger rod 41 in the axial direction by allowing the protrusion 36 of the finger grip 30 in Fig.3 to move in the first guide groove 44.

As shown in Fig.4, Fig.5(C) and Fig.6(A), a wedge shape section 46 (a portion formed in a wedge shape in the cross-section by having an inclined surface rising from the bottom 51 of the first guide groove 44 toward the plunger rod surface 52 while going toward the end edge of the plunger rod 41) is formed in the vicinity of the end edge of the first guide groove 44. As described above, the first guide groove 44 includes a part having a depth in which the protrusion 36 can move without deformation and a wedge shape section 46 in which the protrusion 36 moves with deformation, and the end point of the first guide groove 44 forms an upright wall 46E of the wedge shape section 46. A third guide groove 47 is formed in a range of the length L3 from the upright wall 46E of the wedge shape section 46 on the extension line of the first guide groove 44. The end point 47E of the third guide groove 47 also forms an upright wall.

The second guide groove 45 has a start point 45B which is firmed at a point which is closer to the end edge side than the upright wall 46E of the wedge shape section 46 and an end point 45E which is formed in the vicinity of the end edge. Therefore, there is an overlap part in the range of an axial length L4 (as shown in Fig.5(C)) from the start point 45B of the second guide groove to the end point 47E of the third guide groove.
A partition wall 53 having a thinner cross-section in the upper part than the lower part is formed between the side surface of the third guide groove 47 and the side surface of the second guide groove 45. The side wall 45S of the second guide groove 45 which is closer to the partition wall 53 starts from the start line 54 and extends toward the end edge of the plunger rod 41. The relationship between the position of the start line 54 and the position of the partition wall 53 is shown in Fig.5(B). The axial length L4 of the partition wall 53 is set to a value which is approximately equal to or slightly larger than the thickness T1 of the protrusion 36 of the finger grip 30. The axial length L3 of the third guide groove 47 is set to approximately double of the axial length L4 of the partition wall 53. The third guide groove 47 is formed at a position which is rotated by 60 degrees in the circumferential direction with respected to the second guide groove 45, and each guide groove is formed as a groove having a fan cross sectional shape. The fan cross sectional shapes of the second guide groove 45 and the third guide groove 47 are arranged to be suitable for guiding the axial direction movement of the plunger rod 41 by the protrusion 36 of the finger grip 30 in Fig.3 which moves in the second guide groove 45 and the third guide groove 47.

As shown in Fig.5(B),(C), the tip of the partition wall 53 has a cross section of an angular shape protruding from the center of the plunger rod 41 is set to be lower than the surface 52 of the plunger rod 41. The thickness of the protrusion 36 of the finger grip 30 in Fig.3 is set to a value which is approximately equal to or slightly smaller than the axial length L4 of the partition wall 53. Therefore, it is configured so that the protrusion 36 moves in the circumferential direction while contacting with the surface of the partition wall 53 and rides over the partition wall 53. As shown in Fig.6(B), the partition wall 53 is formed between the start point 45B of the second guide groove 45 and the end point 47E of the third guide groove 47, and it is arranged so that the protrusion 36 can move from the third guide groove 47 to the second guide groove 45.

The inner diameter of the syringe barrel 20, the length of the syringe barrel 20, the axial length of the piston 50, the length L5 between the tip and the end point 47E in the syringe plunger 40, the thickness T1 (the thickness of the protrusion 36) of the flat plate section 31 of the finger grip 30 as an example are set so that the amount of the internal-use solution 60 accommodated in the syringe barrel 20 becomes a predetermined amount when the finger grip 30 is assembled together with the syringe plunger 40 as shown in Fig.1, the syringe plunger 40 is pushed into the syringe barrel 20 and the protrusion 36 of the finger grip 30 reaches the end point 47E of the third guide groove. The length L2 of the second guide groove is set so that the total amount of the internal-use solution 60 in the syringe barrel 20 is administered when the protrusion 36 reaches the end point 45E of the second guide groove 45.

Next, the operation of the finger grip 30 and the syringe plunger 40 configured as described above will be explained.

First, a syringe, in which the syringe barrel 20 is filled with the internal-use solution 60, is prepared. An amount which is approximately equal to or slightly more than the predetermine amount of the solution 60 is put in the syringe barrel 20 from the opening by arranging the syringe barrel 20 shown in Fig.2 so that the syringe barrel 20 stands while putting the flange section 23 up. Next, the piston 50 is put into the syringe barrel 20 from the opening of the flange section 23. As shown in Fig.1(B), the finger grip 30 is attached to the flange section 23 by aligning the notched portion 33 with the flange section 23 and pushing the finger grip 30 against the flange section 23 from the horizontal direction while inserting the flange section 23 between the flat plate section 31 and the cover section 32. By this operation, the opening of the flange section 23 is covered by the flat plate section 31 having the hole 35.

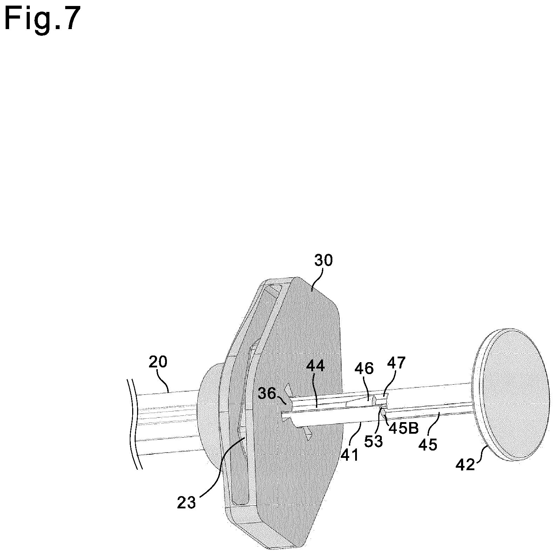

The plunger rod 41 is inserted in the hole 35 from the tip 43 by aligning the protrusion 36 is aligned with the first guide groove 44 so that the protrusion 36 is inserted in the first guide groove 44. Bu this operation, as shown in Fig.7, the finger grip 30 is attached to the flange section 23 of the syringe barrel 20, and the syringe plunger 40 is inserted into the hole 35.

By pushing the plunger head 42, the plunger rod 41 is sent toward the tip of the syringe barrel 20 passing through the hole 35 while the movement in the circumferential direction is restricted by the first guide groove 44 and the protrusion 36. When the protrusion 36 proceeds on the inclined surface of the wedge shape section 46 toward the surface 52 from the bottom 51 of the first guide groove 44, the tip portion of the protrusion 36 is bent by being pushed against the inclined surface, the bent protrusion 36 returns to the original shape after riding over the upright wall 46E which is the end point of the wedge shape section 46. As the root portion of the protrusion 36, namely the flat plate section 31 of the finger grip 30, is ahead of the tip portion of the protrusion 36 when the tip portion of the protrusion 36 which is bent and positioned backward is positioned just above the upright wall 46E, the root portion of the protrusion 36 is advanced to a position above the third guide groove 47. As the axial length L3 of the third guide groove 47 is set to be larger than the thickness T1 of the protrusion 36, for example double of the thickness T1 of the protrusion 36, the advanced flat plate section 31 is still within the range of the third guide groove 47 when the protrusion 36 returns to the original shape. In this way, it is arranged so that the flat plate section 31 does not go beyond the end point 47E of the third guide groove 47.

The air in the barrel is discharged while pushing the excess amount of the internal-use solution 60 out from the injection nozzle section of the syringe barrel 20 in accordance with the axial movement of the plunger rod 41 until when the protrusion 36 reaches the end point 47E of the third guide groove 47.

When the protrusion 36 moves in the third guide groove 47 after riding over the wedge shape section 46 and reaches the end point (the upright wall) 47E, the axial movement of the plunger rod 41 stops and just a predetermined amount of the internal-use solution 60 is kept inside the syringe barrel 20. When the protrusion 36 is positioned inside the third guide groove 47, the movement of the protrusion 36 in the axial direction of the plunger rod 41 is restricted by the upright wall 46E and the end point (the upright wall )47E. Therefore, the plunger rod 41 neither can be pulled out from the syringe barrel 20 nor can be pushed further into the syringe barrel 20. By the above-mentioned procedure, it is possible to provide a syringe having a dose accuracy (accuracy in filling and administration) function in which the syringe barrel 20 is filled with a predetermined amount of the internal-use solution 60. In the operation of pushing the plunger rod 41 into the syringe barrel 20, it causes a clicking sound when the protrusion 36 climbed over the upright wall 46E which is the endpoint of the wedge shape section 46. By this operation, a medical worker will realize that a predetermined amount operation (a stroke) is completed by not only a visual observation but also a sound or a feeling which is transmitted to the hand.

Next, an operation for conducting an injection will be explained. In Fig.7, when the plunger head 42 is rotated by approximately 60 degrees clockwise, the protrusion 36 rides over the partition wall 53, and the protrusion 36 moves into the second guide groove 45. After this operation, the internal-use solution 60 is discharged from the injection nozzle section 22 of the syringe barrel 20 by pushing the plunger head 42. When the protrusion 36 reaches the end point 45E, the total amount of the predetermined amount of the internal-use solution 60 is administered. Even if it is attempted to pull the plunger rod 41 out from the syringe barrel 20 after completing the injecting operation, the protrusion 36 stops at the start point 45B of the second guide groove 45, and the plunger rod 41 cannot be further pulled out from the syringe barrel 20. Even if the plunger head 42 is rotated by approximately 60 degrees counter-clockwise and returns the protrusion 36 to the third guide groove 47, the protrusion 36 cannot ride over the upright wall 46E toward the tip, the movement of the protrusion 36 is obstructed and the plunger rod 41 is prevented from pulling out from the syringe 20.

According to the aforementioned embodiment, it is possible to provide a syringe plunger and a finger grip having both a dose accuracy function and a pulling out prevention function in a medical disposal syringe. A syringe plunger and a finger grip according to the present embodiment are preferable for a prefilled syringe in which a medical solution is previously filled in a syringe barrel and assembled together.

### Explanation of References

- 10: syringe
- 20: syringe barrel
- 21: barrel section
- 22: injection nozzle section
- 23: flange section
- 30: finger grip
- 31: flat plate section
- 32: cover section
- 33: notched portion
- 34: internal space
- 35: hole
- 36: protrusion
- 40: syringe plunger
- 41: plunger rod
- 42: plunger head
- 43: tip
- 44: first guide groove
- 45: second guide groove
- 45B: start point
- 45E: end point
- 45S: side wall
- 46: wedge shape section
- 46E: upright wall
- 47: third guide groove
- 47E: end point
- 51: bottom
- 52: surface
- 53: partition wall
- 54: start line
- 50: piston
- 60: internal-use solution

## Claims

1. A medical syringe plunger comprising a plunger rod and a plunger head which is formed at the end edge of the plunger rod, for using by being inserted into a hole of a finger grip which is to be attached to a flange section of a syringe barrel, wherein
the plunger rod includes a first guide groove and a second guide groove for guiding the axial movement by a protrusion of a finger grip which moves in the guide grooves; the first guide groove is formed in a range of a first predetermined length in the axial direction from the tip;
the second guide groove is formed in a range of a second predetermined length in the axial direction at a position on the plunger rod which is rotated by a predetermined angle in the circumferential direction with respect to the first guide groove; and
the protrusion is formed to extend toward the center of the hole of the finger grip.

2. A medical syringe plunger according to Claim 1, wherein
a protrusion transition section is formed between the first guide groove and the second guide groove, the protrusion transition section enables the protrusion to transit from the first guide groove to the second guide groove.

3. A medical syringe plunger according to Claim 1 or 2, further comprising
an axial direction movement restriction section for allowing the protrusion to move toward the plunger head until the end point of the first guide groove and restricting the protrusion's movement in the axial direction on the extension line of the first guide groove after the protrusion passes over the end point, wherein
the second guide groove has a start point formed at a position which is not closer to the tip side than the axial direction movement restriction section and an end point which is formed at the side of the end edge.

4. A medical syringe plunger comprising a plunger rod and a plunger head which is formed at the end edge of the plunger rod, for using by being inserted into a hole of a finger grip which is to be attached to a flange section of a syringe barrel, wherein
the plunger rod includes a first guide groove for guiding the axial movement by a protrusion of a finger grip which moves in the guide groove;
the first guide groove is formed in a range of a first predetermined length in the axial direction from the tip;
the protrusion is formed to extend toward the center of the hole of the finger grip; and the first guide groove includes a portion formed in a wedge shape having an inclined plane, the inclined plane starts from the bottom of the first guide groove and goes toward the surface of the plunger rod in the part between a point before the end point and the end point, and the end point is formed as an upright wall.

5. A medical finger grip having a shape for attaching to a flange section of a syringe barrel to cover at least a part of the flange section, wherein
the finger grip has a flat plate section for covering an opening of the flange section, the flange section has a hole through which a plunger rod passes, and the flat plate section has a protrusion which extends toward the center of the hole; and
the protrusion has a length which reaches a predetermined depth in the guide grooves which are formed in the plunger rod, and the protrusion is formed so that the tip is bent when the protrusion rides over a structure and returns to the original shape after the protrusion passed over the structure.
